# EUROPEAN PATENT SPECIFICATION

(11) **EP 1 290 985 B1**
(45) Date of publication and mention of the grant of the patent: **09.04.2008**
(21) Application number: 02018509.6
(22) Date of filing: 16.08.2002
(51) Int. Cl.: A61F 2/44

(54) **Intersomatic cage for posterior fusion surgery to the lumbar column**
Zwischenwirbelkäfig für Operationen mit posteriorem Zugang zur Wirbelsäule
Cage intersomatique pour intervention chirurgicale postérieure au rachis lombaire

(30) Priority: 16.08.2001 ES 200101905
(43) Date of publication of application: 12.03.2003
(60) Divisional application: 07019647.2
(73) Proprietor: Biomet Spain Orthopaedics S.L., Paterna (Valencia) (ES)
(72) Inventor: Lopez Casquero, Carlos, 46988 Fuente del Jarro (ES); Sierrra Aparici, Alfredo, 46988 Fuente del Jarro (ES)
(74) Representative: Koepe, Gerd L.

(56) References cited:
- WO-A-02/17823
- FR-A- 2 727 004
- US-A- 4 349 921
- US-A- 4 834 757
- US-A- 6 136 001
- US-A- 6 159 211
- US-A- 6 159 244
- US-B1- 6 371 988

## Description

### Object of the invention

This invention refers to an intersomatic cage for posterior fusion surgery to the lumbar column and for surgery involving the insertion of a transforaminal implant, which represents solutions in terms of improving the vertebral fusion of the lumbar column within the scope of the surgical procedures referred to as PLIF (Posterior Lumbar Interbody Fusion) or TLIF (Transforaminal Lumbar Interbody Fusion), by means of:
I) A monobloc intersomatic cage;
II) an expansive intersomatic cage.

The function of these intersomatic cages is as an element acting as a spacer between and holding the lumbar vertebrae between which they are placed, in such a way that they prevent their collapse and increase the initial mechanical rigidity during lumbar fusion. At the same time, these intersomatic cages serve as a support and structural improvement in combination with other types of instrumentation, such as bars and plates joined to transpedicular screws or laminar and pedicular hooks.

### Background to the invention

The Posterior Intersomatic Fusion of the Lumbar Column or, as it is better known, Posterior Lumbar Interbody Fusion (PLIF), was initially developed by Cloward in the 1940s to treat lumbar pain due to intervertebral hernias or the degeneration of disks. In this surgical technique, the central portion of the disk is removed and replaced by multiple blocks of osseous graft from the patient himself or by artificial bone. More recently, pedicular screws and plats have been used to treat the same degenerative conditions. In 1988, Dr. Steffe recommended the use of these systems of fixing using pedicular screws in combination with these grafts, with the aim of better distributing the loads with the anterior part of the lumbar column. Nevertheless, problems have still arisen with the transplanted bone, such as the collapse of the graft due to the insufficient initial mechanical resistance and due to biological incompatibility in cases in which artificial bone had been grafted. Currently, other variants of posterior approach have been developed, such as the transforaminal route (TLIF), in which a posterolateral surgical approach is made along a transforaminal route, but which is basically the same technique as the PLIF.

In this day and age, the intersomatic cages, as they are described in this invention, resolve a high percentage of diseases related to the dorsolumbar vertebral disks. The principal surgical indications for these are:
- Damage to the disks due to traumatisms, tumours or degeneration of the vertebrae, for which decompression is necessary, as is restoring the original height of the intervertebral space by means of arthrodesis (osseous fusion).
- Chronic lumbago related to the vertebral disks, for which all the conservative treatments such as stretching exercises, analgesics, muscular relaxants etc., do not give positive results.
- It is used as an adjunct in transpedicular surgery and surgery with bars or plates, thereby increasing the rigidity of the system implanted.

The problems that are currently being created, and that are in part still not resolved for the majority of implants, are as follows:
- Incomplete fusion due in part to the actual design of the implant.
- Displacement of the dura mater with the consequent risk of neurological lesions, again due to the design of the implant and its instrumentation.
- Radiopaque materials and massive designs that do not permit verification, during the post-operative period, of whether a correct osseous fusion exists.

### Description of the invention

With the intersomatic cage for posterior fusion surgery to the lumbar column and for surgery involving the insertion of an implant, which is the object of this invention, it is sought to avoid or to palliate all of these difficulties. For this purpose, these cages are designed with the aim of providing initial mechanical resistance prior to fusion, of facilitating fusion through osseous growth through the windows that it disposes of in its design, into which autologous graft (graft from the patient himself) is inserted, and finally, of facilitating the post-operative follow-up of osseous growth through the windows or holes provided for in its design and/or use of radiotransparent materials (materials that permit the osseous growth to be scanned by X-rays), such as for example: PEEK (polyether ether ketone), Carbon Fibre or else PEEK-based Composites of Carbon Fibre.

The invention relates to an intersomatic cage for posterior fusion surgery to the lumbar column of the type that uses preferably intersomatic cages in order to restore the height of the intersomatic gap between the lumbar vertebras- wherein the cage is a monobloc cage and comprises
- lateral windows that allow radiological control of bone growth;
- one vertical trough;
- said lateral windows and trough forming a cavity adapted to receive a bone implant for the fusion or arthrodesis;
- the cage having saw-shaped teeth or harpoon-shaped teeth on its top and bottom side only intended to increase the holding ability and the support surface between the adjacent vertebras;
- a coupling system being available at the back side of the cage to introduce the implant in place by using the appropriate instrumentation; the geometrical design of the cage being of an essentially oval profile in order to couple with the general anatomy improving the contact area, the load distribution in the implant, to improve bone fusion and to maintain itself attached to the vertebras by the means of saw-shaped or harpoon-shaped teeth on the top and bottom of the cage that prevent the expulsion of the cage implant, and to an intersomatic cage for posterior fusion surgery to the lumbar column of the type that uses preferably intersomatic cages in order to restore the height of the intersomatic gap between the lumbar vertebras, wherein the cage is an expandable cage and comprises
- titled lateral windows that allow radiological control of bone growth;
- one vertical trough;
- said lateral windows and trough forming a cavity adapted to receive a bone implant for the fusion or arthrodesis;
- the cage having saw-shaped teeth or harpoon-shaped teeth on its top and bottom side only intended to increase the holding ability and the support surface between the adjacent vertebras;
- a coupling system being available at the back side of the cage to introduce the implant in place by using the appropriate instrumentation;
the geometrical design of the cage being of an essentially oval profile in order to couple with the general anatomy improving the contact area, the load distribution in the implant, to improve bone fusion and to maintain itself attached to the vertebras by the means of saw-shaped or harpoon-shaped teeth on the top and bottom of the cage that prevent the expulsion of the cage implant, and a slidable washer inside, said cavity being capable of causing the cage to expand due to the tilt of the tilted lateral windows.

The intersomatic cages for lumbar fusion surgery to which the invention refers are based on a system of essentially hollow intersomatic cages that come in two variants, all of which perform the same function and which are described in further detail below.

### MONOBLOCK CAGE

This monoblock cage is an intersomatic separating device for lumbar column vertebrae. It is used to restore the natural height of the disc and is used as a bridge for bone fusion. It is composed of a cage with two side windows and one vertical window that form the hollow space required to hold the bone graft that will form the fusion by growing the bone between the adjoining vertebrae. It is an essentially oval-shaped section, thus optimising the implant's contact with the bone so that it adapts to the discs in the sub-chondral zone of the vertebrae to be fused. Its distal part is also higher to maintain the natural curvature or lordosis. The distal part is rounded to facilitate its implantation without damaging nerve roots or soft spots. The top and bottom of the cage are equipped with a tilted sawtooth section with the aim to: 1) prevent the device from being expelled by a harpoon or blocking effect; 2) prevent the vertebrae from collapsing as a result of being crushed by increasing the surface area of contact with the bone; and 3) preventing any movement which impedes the proper growth of the bone between the cage and the adjacent vertebrae.

### EXPANSION CAGE

As in the case of the monoblock cage described above, the second type consists of an intersomatic separating device for lumbar column vertebrae. The applications and advantages are the same, however, the aim of this invention is to augment the fastening power of the device to the intersomatic space while maintaining the curvature or lordosis of the lumbar column after the cage expands. Its principal advantage over the other designs is the ease with which it is implanted, since the height of the distal part of the cage is initially lower before it expands once implanted, thus making implantation less traumatic.

It has a sliding washer inside which, in order to be applied, once the cage is in position, an instrument is used to push the washer against the cage, causing the distal end to expand due to the tilt of the lateral window, which serves an angulated lateral guide. The lateral window is also equipped with a stopper or projection that limits the washer's movement and prevents it from leaving the cage.

### Description of the Drawings

To supplement the description provided herein, and for a better understanding of the characteristics of the invention, this description is accompanied by a series of figures which represents the following, on an illustrative but non-limitative basis:
Figure 1 represents a side view of the final assembly of a practical execution of the monoblock intersomatic cage to which the invention refers, relative to the placement of the cage in the intersomatic channel.
Figure 2 represents a rear view of the same assembly, relative to the placement of two intersomatic cages and bone graft from a rear view.
Figure 3 shows a view of the monoblock intersomatic cage from an isometric perspective.
Figure 4 shows a rear view of the monoblock intersomatic cage.
Figure 5 shows a side view of the monoblock intersomatic cage.
Figure 6 shows an overhead view of the monoblock intersomatic cage.
Figure 7 represents a side view of the final assembly of a practical execution of the expansive intersomatic cage to which the invention refers, relative to the placement of the cage in the intersomatic channel.
Figure 8 represents a rear view of the same assembly, relative to the placement of two expansive intersomatic cages and bone graft from a rear view.
Figure 9 shows a view of the expansive intersomatic cage from an isometric perspecti ve.
Figure 10 shows a rear view of the expansive intersomatic cage.
Figure 11 shows a side view of the expansive intersomatic cage.
Figure 12 shows a view from an isometric perspective of washer in the expansive intersomatic cage.
Figure 13 shows an overhead view of the expansive intersomatic cage
Figure 14 shows a view of the expansive intersomatic cage in the expanded position.

### Preferred Embodiment of the Invention

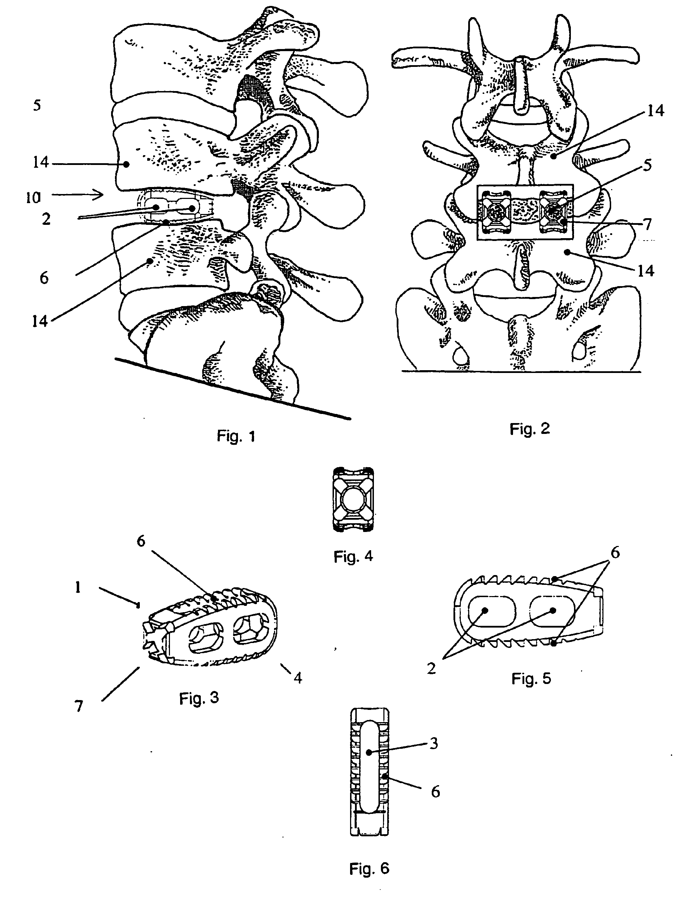

As seen in the attached figures (Figures 1 through 6) the monoblock intersomatic cage 1 is received between two lumbar vertebrae 14 (Figures 1 and 2). The PLIF technique provides for the symmetrical and parallel placement of two cages 1 in each intersomatic space.

This cage comprises side windows 2 and a vertical pass-through window 3, these windows forming the recess 4 in which the bone graft 5 will be received for fusion, or arthrodesis. It is possible to obtain such graft from the patient himself/herself or from a bone bank, or to use a synthetic material. The cage 1 is provided with serrated teeth 6 at both its upper and lower portions, aiming at enhancing the supporting capacity and bearing surface between adjacent vertebrae 14. It is also provided with a coupling system 7 on its back part to introduce the implant by means of the suitable instrument. The sectional geometrical design is essentially oval shaped, so that it can conform the anatomy of the vertebral subcondral disks 14 and keep the natural lordosis or angulation of the spinal column at lumbar level. The correct size will be selected according to the conditions of each case, all cages having identical geometrical characteristics so as to fit any intervertebral size. In addition they will be based on anthropometric studies.

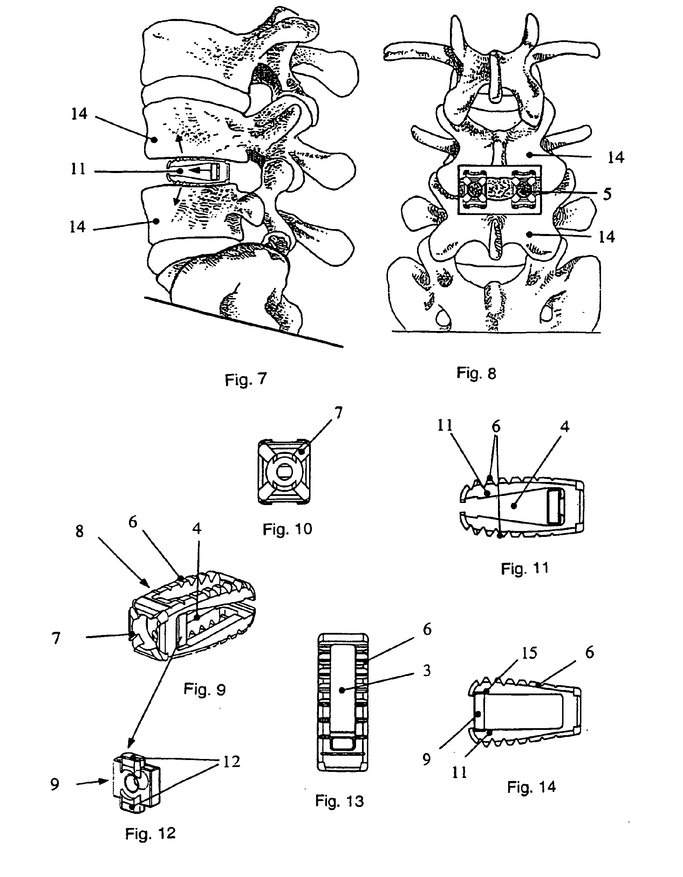

Figures 7 through 17 show the geometrical characteristics of the expansive intersomatic cage 8 and its situation in the surgical field. Like in the case of the single block intersomatic cage, two symmetrical cages are placed in parallel in the intersomatic space existing between two lumbar vertebrae 14 (Figures 7 and 8).

This element comprises a cage 8 that is open at its front part, and an internal washer 9 previously mounted in the inner part of the cage, the washer being able to be slid along an inner canal 4 of the cage, which will be provided with a suitable slope and able to expand the cage 8 at the open or front part, by sliding until it encounters a butt 15 of the cage. At the same time, the washer 9 is guided by some salients or ridges 12 located on another vertical window 3 arranged in the cage 8. The washer sliding motion 9 is made by the suitable instruments once the cage has been introduced in its final position. This cage of the invention is also provided with a few serrated teeth 6 on the upper and lower surfaces of the cage 8 serving the same purpose as that of the monoblock cage. At the same time, there is provided a back coupling system 7 to introduce the cage 8 next to the washer 9 together.

That is, when this cage 8 is at its standing position (Figure 11), the height of the open end is smaller than that of the closed end. However, when the washer 9 is made to slide at the open end on its butts 15, the heights are inverted, being the height of the open end with the washer 9 larger than the height of the cage closed end 8. In any case, the height of the open end is larger when the washer 9 is present at this end, than when said washer is in the opposite end, thus modifying the cage angle.

The application and functioning are very simple. For that purpose, upon identifying the vertebrae 14 to be intervened by applying the corresponding technique, the intersomatic cage is placed between both vertebrae, with their recess 4 being filled with bone material 5. When introducing the cage, the coupling system 7 will be employed as to have the serrated teeth 6 or harpoon make the introduction - but not involuntary removal thereof - easier, as these teeth tend to stick into the subcondral bone in the presence of removal motion, thus enhancing the prosthesis fixation by avoiding expulsion thereof.

As the single block intersomatic cage 1 is introduced in its corresponding place, and because of its angles and oval design, it separates the vertebrae 14 between which it should be placed, so as to follow the general vertebral anatomy, improve the contact surface and therefore, the distribution of the implant loads. In addition, it keeps the natural angulation or lordosis of the spinal cord at these lumbar levels.

The contact between the bone graft 5 and the body of the adjacent vertebrae 14 is attained by the side 2 and upper 3 windows, which helps the osteointegration thus enhancing prosthesis fixation and avoiding failures. At the same time and thanks to these windows on the body 1 and in certain cases, because of the material used for these cages, e.g. the PEEK, examination of the implant is made easier by means of radiographic techniques or the like. Since they are not radio-opaque materials, visibility is possible to better control its evolution.

On the other hand, the variant of the expansive intersomatic cage has some features of its own in terms of structure, as well as some advantages, too. The application method of this cage is the same as that of the monoblock cage. In order to place it, the washer 9 is located in its standing position (Figure 11), so that the cage body inclination 8 facilitates introduction in the intervertebral space. Upon completion, this washer 9 is moved into its working position by applying the corresponding method (Figure 14).

The washer 9 is moved along the cavity 4, the window 3 serving as a guide, since the salients or ridges 12 are located in the inner part so that sliding along this window is allowed with no transversal movements hindering such sliding motion. At the end of this path, there is a stop 15 against which the washer stays at its working position, thus avoiding back collapse of the cage and the expulsion of the washer itself.

In this situation, the cage body 8 has changed the slope of its inclination by inverting it, so that the spinal cord angulation or lordosis is achieved once the washer 9 has been moved into its final position. This cage is also provided with serrated teeth 6 which, like the at the monoblock cage, prevent involuntary removal because they stick into the vertebra body 14. It is also provided with a cavity 4 for the insertion of bone material 5, and a window 3 which makes the bone fusion with adjacent vertebrae easier.

Now that the nature of this invention and a way of putting it into practice have been described, we should add that changes can be made to the shape, materials and arrangement of the invention and its components as long as these alterations do not substantially change the characteristics of the invention described in the following claims.

## Claims

1. An intersomatic cage for posterior fusion surgery to the lumbar column of the type that uses preferably intersomatic cages (1) in order to restore the height of the intersomatic gap between the lumbar vertebras (14), wherein the cage is a monoblock cage (1) and comprises
- lateral windows (2) that allow radiological control of bone growth;
- one vertical trough (3);
- said lateral windows (2) and trough (3) forming a cavity (4) adapted to receive a bone implant (5) for the fusion or arthrodesis;
- the cage (1) having saw-shaped teeth or harpoon-shaped teeth (6) on its top and bottom side only intended to increase the holding ability and the support surface between the adjacent vertebras (14);
- a coupling system (7) being available at the back side of the cage (1) to introduce the implant in place by using the appropriate instrumentation;
- the geometrical design of the cage (1) being of an essentially oval profile in order to couple with the general anatomy improving the contact area, the load distribution in the implant, to improve bone fusion and to maintain itself attached to the vertebras (14) by the means of saw-shaped or harpoon-shaped teeth (6) on the top and bottom of the cage (1) that prevent the expulsion of the cage implant.

2. An intersomatic cage for posterior fusion surgery to the lumbar column of the type that uses preferably intersomatic cages (8) in order to restore the height of the intersomatic gap between the lumbar vertebras (14), wherein the cage (8) is an expandable cage and comprises
- tilted lateral windows (2) that allow radiological control of bone growth;
- one vertical trough (3);
- said tilted lateral windows (2) and trough (3) forming a cavity (4) adapted to receive a bone implant (5) for the fusion or arthrodesis;
- the cage (8) having saw-shaped teeth or harpoon-shaped teeth (6) on its top and bottom side only intended to increase the holding ability and the support surface between the adjacent vertebras (14);
- a coupling system (7) being available at the back side of the cage (8) to introduce the implant in place by using the appropriate instrumentation;
- the geometrical design of the cage (8) being of an essentially oval profile in order to couple with the general anatomy improving the contact area, the load distribution in the implant, to improve bone fusion and to maintain itself attached to the vertebras (14) by the means of saw-shaped or harpoon-shaped teeth (6) on the top and bottom of the cage (8) that prevent the expulsion of the cage implant; and
- a slidable washer inside said cavity being capable of causing the cage to expand due to the tilt of the tilted lateral windows (2).

3. The intersomatic cage according to claim 1 or claim 2, wherein the overall profile of the cage (1, 8) is angulated, maintaining the lordosis or the natural angulation of the intervened lumbar gaps.

4. The intersomatic cage according to claim 2, wherein the expandable cage (8) has a lateral angulated guide (11) and a vertical guide (3), through which said internal washer (9) is slided along.

5. The intersomatic cage according to claim 2 or claim 4, wherein the expansive intersomatic cage (8) has a protrusion or step (16) for the fixation of the washer (9) inside the cage (8).

6. The intersomatic cage according to any of claims 2 and 4 to 5, wherein the washer (9) has some salients (12) that remain lodged inside the trough (3), in a way that it will allow sliding along this trough serving as a guide.

7. The intersomatic cage according to any of claims 2 and 4 to 6, wherein the angulation of the lateral guide (11) is such that the cage (8) adopts a slope when the washer (9) is in its initial or rest position, changing this slope when the washer is in its working position.

8. The intersomatic cage according to any of claims 2 and 4 to 7, wherein the expansive intersomatic cage (8), with the washer (9) located in its working position on its protrusions (15), falls upon the slope in the vertical direction.

9. The intersomatic cage according to any of claims 2 and 4 to 8, wherein the expansive intersomatic cage (8), with washer (9) located in its working position on its protrusions (15), modifies the slope of the cage (8) in the vertical direction of widening, wherein the height of the open end is larger when the washer (9) is at this end than when it is on the opposite end.

## Patentansprüche

1. Zwischenwirbel-Käfig für Operationen mit Posterior-Zugang zur Wirbelsäule von dem Typ, der vorzugsweise von Zwischenwirbel-Käfigen (1) Gebrauch macht, um die Höhe des Zwischenwirbel-Abstandes zwischen den Lendenwirbeln (14) wieder herzustellen, worin der Käfig (1) ein Monoblock-Käfig ist und umfasst:
- seitliche Fenster (2), die eine radiologische Kontrolle des Knochenwachstums erlauben;
- ein vertikale Rinne (3);
- wobei die seitlichen Fenster (2) und die Rinne (3) einen Hohlraum (4) bilden, der dafür geeignet ist, ein Knochen-Implantat (5) für die Verschmelzung oder Arthrodese aufzunehmen;
- wobei der Käfig (1) sägeförmige Zähne oder harpunenförmige Zähne (6) nur auf seiner Ober- und Unterseite aufweist, mit denen es beabsichtigt ist, das Haltevermögen und die Stützfläche zwischen den benachbarten Wirbeln (14) zu erhöhen;
- ein Kopplungssystem (7), das auf der Rückseite des Käfigs (1) verfügbar ist, um das Implantat unter Verwendung der passenden Instrumente an Ort und Stelle einzusetzen;
- wobei das geometrische Design des Käfigs (1) von im Wesentlichen ovalem Profil ist, um mit der allgemeinen Anatomie unter Verbesserung der Kontaktfläche und der Ladungsverteilung in dem Implantat zu koppeln, die Verschmelzung der Knochen zu verbessern und sich selbst mit den Wirbeln (14) mittels der sägeförmigen oder harpunenförmigen Zähne (6) auf der Ober- und Unterseite des Käfigs (1) verbunden zu halten die das Ausstoßen des Käfig-Implantats verhindern.

2. Zwischenwirbel-Käfig für eine Operation mit Posterior-Zugang zur Wirbelsäule von dem Typ, der vorzugsweise von Zwischenwirbel-Käfigen (8) Gebrauch macht, um die Höhe des Zwischenwirbel-Abstands zwischen den Lendenwirbeln (14) wieder herzustellen, worin der Käfig (8) ein expandierbarer Käfig ist und umfasst:
- gekippte seitliche Fenster (2), die eine radiologische Kontrolle des Knochenwachstums erlauben;
- eine vertikale Rinne (3);
- wobei die gekippten seitlichen Fenster (2) und die Rinne (3) einen Hohlraum (4) bilden, der dafür angepasst ist, ein Knochen-Implantat (5) für die Verschmelzung oder Arthrodese aufzunehmen;
- wobei der Käfig (8) sägeförmige Zähne oder harpunenförmige Zähne (6) nur auf seiner Oberseite und seiner Unterseite aufweist, mit denen es beabsichtigt ist, das Haltvermögen und die Stützoberfläche zwischen benachbarten Wirbeln (14) zu erhöhen;
- wobei ein Kopplungssystem (7) auf der Rückseite des Käfigs (8) verfügbar ist, um das Implantat unter Verwendung der passenden Instrumente an Ort und Stelle einzuführen;
- wobei das geometrische Design des Käfigs (8) von im Wesentlichen ovalem Profil ist, um mit der allgemeinen Anatomie unter Verbesserung der Kontaktfläche und der Ladungsverteilung des Implantats zu koppeln, um eine Knochenverschmelzung zu verbessern und sich selbst mit den Wirbeln (14) mittels der sägeförmigen oder harpunenförmigen Zähne (6) auf der Oberseite und der Unterseite des Käfigs (8) verbunden zu halten, die das Ausstoßen des Käfig-Implantats verhindern; und
- eine gleitfähige Distanzscheibe innerhalb des Hohlraums, die in der Lage ist, dafür zu sorgen, dass sich der Käfig aufgrund der Kippung der gekippten seitlichen Fenster (2) ausdehnt.

3. Zwischenwirbel-Käfig nach Anspruch 1 oder Anspruch 2, worin das Gesamtprofil des Käfigs (1, 8) geneigt ist, wobei die Lordose oder natürliche Neigung der dazwischen gelegenen Lendenwirbel-Spalte aufrechtzuerhalten.

4. Zwischenwirbel-Käfig nach Anspruch 2, worin der expandierbare Käfig (8) eine seitliche geneigte Führung (11) und eine vertikale Führung (3) aufweist, über die die innere Distanzscheibe (9) entlang gleiten kann.

5. Zwischenwirbel-Käfig nach Anspruch 2 oder Anspruch 4, worin der ausdehnbare Zwischenwirbel-Käfig (8) einen Vorsprung oder eine Stufe (16) für die Befestigung der Distanzscheibe (9) innerhalb des Käfigs (8) aufweist.

6. Zwischenwirbel-Käfig nach einem der Ansprüche 2 und 4 bis 5, worin die Distanzscheibe (9) einige Vorsprünge (12) aufweist, die innerhalb der Rinne (3) in einer Weise festgeklemmt bleiben, dass dies ein Gleiten entlang dieser Rinne erlaubt, die als Führung dient.

7. Zwischenwirbel-Käfig nach einem der Ansprüche 2 und 4 bis 6, worin die Neigung der seitlichen Führung (11) derart ist, dass der Käfig (8) eine Neigung annimmt, wenn die Distanzscheibe (9) in ihrer anfänglichen oder Ruhe-Position ist, wobei er diese Neigung ändert, wenn die Distanzscheibe in ihrer Arbeitsposition ist.

8. Zwischenwirbel-Käfig nach einem der Ansprüche 2 und 4 bis 7, worin der expandierbare Zwischenwirbel-Käfig (8) dann, wenn die Distanzscheibe (9) in ihrer Arbeitsposition auf ihren Vorsprüngen (15) angeordnet ist, in der vertikalen Richtung auf die Neigung fällt.

9. Zwischenwirbel-Käfig nach einem der Ansprüche 2 und 4 bis 8, worin der expandierbare Zwischenwirbel-Käfig (8) dann, wenn die Distanzscheibe (9) in ihrer Arbeitsposition auf ihren Vorsprüngen (15) angeordnet ist, die Neigung des Käfigs (8) in der vertikalen Richtung eines Aufweitens modifiziert, worin die Höhe des offenen Endes größer ist, wenn die Distanzscheibe (9) an diesem Ende ist, als wenn sie in dem gegenüberliegenden Ende ist.

## Revendications

1. Cage intersomatique pour une chirurgie de fusion postérieure de la colonne lombaire du type utilisant préférentiellement des cages intersomatiques (1) permettant de restaurer la hauteur de l'espace intersomatique entre les vertèbres lombaires (14), dans laquelle la cage (1) est une cage monobloc et comprend:
- des fenêtres latérales (2) qui permettent un contrôle radiologique de croissance osseuse ;
- une ouverture oblongue verticale (3) ;
- lesdites fenêtres latérales (2) et ladite ouverture oblongue (3) formant une cavité (4) adaptée à recevoir un implant osseux (5) pour la fusion ou arthrodèse ;
- la cage (1) ayant des dents (6) en forme de scie ou en forme de harpon sur ses côtés de dessus et de dessous uniquement destinées à augmenter la capacité de maintien et la surface de support entre les vertèbres adjacentes (14) ;
- un système (7) d'accouplement étant disponible au niveau du côté arrière de la cage (1) pour introduire l'implant en place en utilisant les instruments appropriés ;
- la conception géométrique de la cage (1) étant d'un profil essentiellement ovale afin de s'harmoniser avec l'anatomie générale améliorant la zone de contact, la répartition de charge dans l'implant, pour améliorer une fusion osseuse et pour se maintenir fixée aux vertèbres (14) au moyen de dents (6) en forme de scie ou en forme de harpon sur le dessus et le dessous de la cage (1) ce qui empêche l'expulsion de l'implant de cage.

2. Cage intersomatique pour une chirurgie de fusion postérieure de la colonne lombaire du type utilisant préférentiellement des cages intersomatiques (8) permettant de restaurer la hauteur de l'espace intersomatique entre les vertèbres lombaires (14), dans laquelle la cage (8) est une cage expansible et comprend :
- des fenêtres latérales inclinées (2) qui permettent un contrôle radiologique de croissance osseuse ;
- une ouverture oblongue verticale (3) ;
- lesdites fenêtres latérales inclinées (2) et ladite ouverture oblongue (3) formant une cavité (4) adaptée à recevoir un implant osseux (5) pour la fusion ou arthrodèse ;
- la cage (8) ayant des dents (6) en forme de scie ou des dents en forme de harpon sur ses côtés de dessus et de dessous uniquement destinées à augmenter la capacité de maintien et la surface de support entre les vertèbres adjacentes (14) ;
- un système (7) d'accouplement étant disponible au niveau du côté arrière de la cage (8) pour introduire l'implant en place en utilisant les instruments appropriés ;
- la conception géométrique de la cage (8) étant d'un profil essentiellement ovale afin de s'harmoniser avec l'anatomie générale améliorant la zone de contact, la répartition de charge dans l'implant, pour améliorer une fusion osseuse et pour se maintenir fixée aux vertèbres (14) au moyen de dents (6) en forme de scie ou en forme de harpon sur le dessus et le dessous de la cage (8) ce qui empêche l'expulsion de l'implant de cage ; et
- une rondelle coulissante à l'intérieur de ladite cavité étant capable d'amener la cage à s'élargir en raison de l'inclinaison des fenêtres latérales inclinées (2).

3. Cage intersomatique selon la revendication 1 ou la revendication 2, dans laquelle le profil général de la cage (1,8) fait un angle, maintenant la lordose ou l'angulation naturelle des espaces lombaires sur lesquels on intervient.

4. Cage intersomatique selon la revendication 2, dans laquelle la cage expansible (8) a un guide latéral (11) qui fait un angle et un guide vertical (3), le long desquels ladite rondelle interne (9) est glissée.

5. Cage intersomatique selon la revendication 2 ou la revendication 4, dans laquelle la cage intersomatique expansible (8) a une saillie ou une marche (16) pour la fixation de la rondelle (9) à l'intérieur de la cage (8).

6. Cage intersomatique selon l'une quelconque des revendications 2 et 4 à 5, dans laquelle la rondelle (9) a des saillies (12) qui restent logées à l'intérieur de l'ouverture oblongue (3) de façon à permettre un coulissement le long de cette ouverture oblongue servant de guide.

7. Cage intersomatique selon l'une quelconque des revendications 2 et 4 à 6, dans laquelle l'angulation du guide latéral (11) est telle que la cage (8) adopte une pente lorsque la rondelle (9) est dans sa position initiale ou de repos, cette pente changeant lorsque la rondelle est dans sa position de travail.

8. Cage intersomatique selon l'une quelconque des revendications 2 et 4 à 7, dans laquelle la cage intersomatique expansible (8), avec la rondelle (9) située dans sa position de travail sur ses saillies (15), suit la pente dans la direction verticale.

9. Cage intersomatique selon l'une quelconque des revendications 2 et 4 à 8, dans laquelle la cage intersomatique expansible (8), avec la rondelle (9) située dans sa position de travail sur ses saillies (15), modifie la pente de la cage (8) dans la direction verticale d'élargissement, dans laquelle la hauteur de l'extrémité ouverte est plus grande lorsque la rondelle (9) est au niveau de cette extrémité que lorsqu'elle est sur l'extrémité opposée.
